# EUROPEAN PATENT APPLICATION

(11) **EP 1 002 798 A1**
(43) Date of publication of application: **24.05.2000**
(21) Application number: 98122031.2
(22) Date of filing: 20.11.1998
(51) Int. Cl.: C07D 487/04, C07D 231/38, C07D 231/40

(54) **An improved process for preparing a therapeutically active pyrazolopyrimidinone derivative**

(71) Applicant: Orchid Chemicals & Pharmaceuticals Ltd., Chennai 600 086 (IN)
(72) Inventor: Chaudhari, Deoram Totaram, Besant Nagar Chennai 600 090 (IN); Deshpande, Pandurang Balwantrao, Indira Nagar, Chennai 600 020 (IN); Khan, Rashid Abdul Rehman K-2 Manek Apartment, Adyar Chennai 600 020 (IN)
(74) Representative: Beneduce, Gianna

(57) **Abstract**

An improved process which allows to obtain 5-[2-ethoxy-5-(4-methylpiperazin-1-ylsulphonyl)phenyl]-1-methyl-3-n.propyl-1,6-dihydro-7H-pyrazolo[4,3-d]pyrimidin-7-one, also known with the generic name sildenafil, in pure form and very good yield and further demontrated to be economically advantageous when compared to the known processes.

## Description

The present invention relates to an improved process for the preparation of a pyrazolopyrimidinone derivative endowed with a remarkable therapeutic activity, i.e. 5-[2-ethoxy-5-(4-methylpiperazin-1-ylsulphonyl)phenyl]-1-methyl-3-n.propyl-1,6-dihydro-7H-pyrazol[4,3-d]pyrimidin-7-one, also known with the generic name sildenafil, having the structure formula:

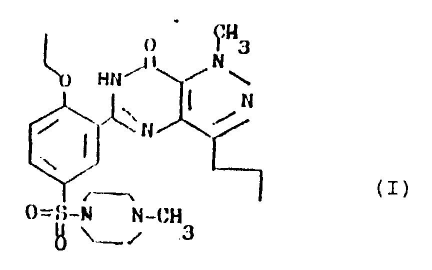

A further aim of the invention is to provide new compounds which are useful intermediates in the process of the present invention.

Sildenafil is described and claimed in USP n°5,250,534 issued on October 5,1993 in the name of the firm Pfizer Inc.: it shows an interesting activity as vasodilator agent and, in particular, through a potent inhibiting effect on specific phosphodiesterases, it has been successfully studied and developed in the treatment of male erectile disfunction.

According to the invention it is provided a process which allows to obtain sildenafil in pure form and with very good yield and further demonstrated to be economically advantageous when compared to the known processes. Known processes for the preparation of sildenafil are described in the above cited USP n°5,250,534 and in EP -A-0812845.

The process of the present invention is carried out following the reaction scheme hereinafter depicted, which also includes alternative routes for preparing specific intermediates therein used:

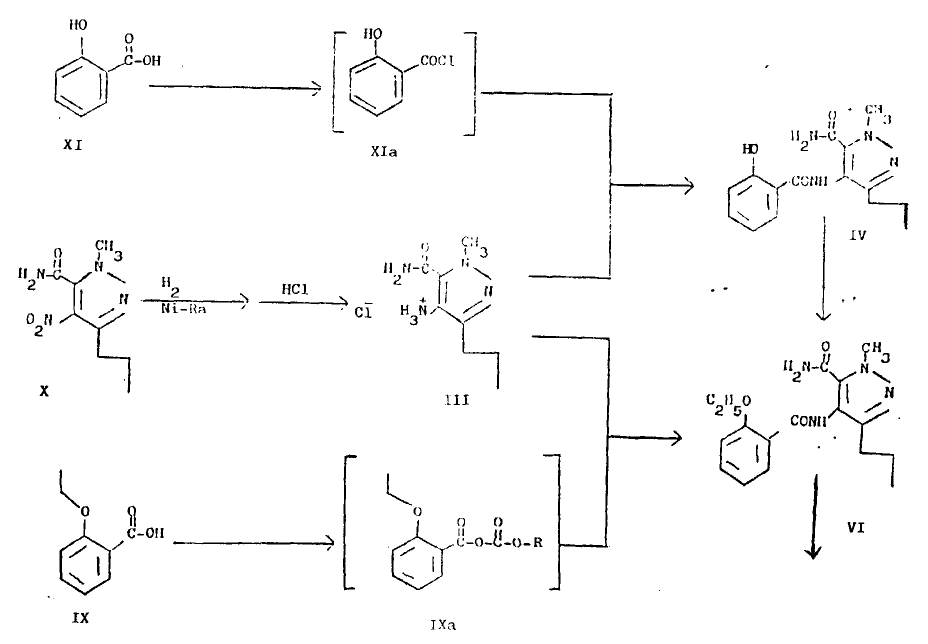

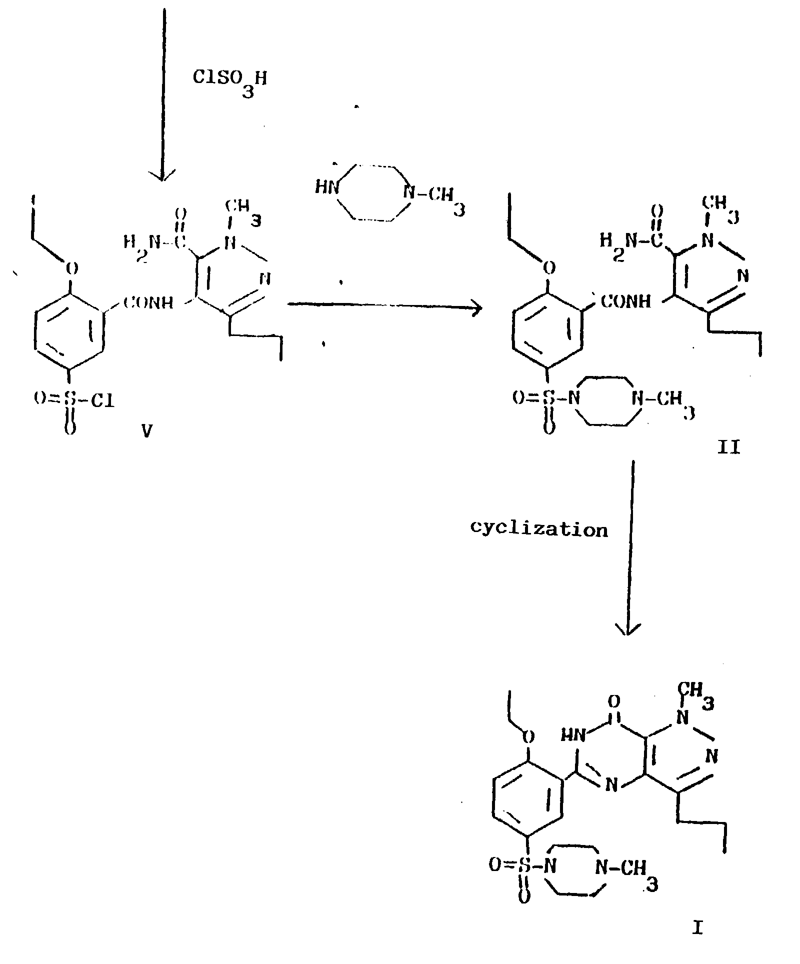

Carefully examining the above scheme it can be noticed that condensation of the mixed anhydride of 2-ethoxybenzoic acid (IXa) (prepared in situ by reaction of compound (IX) with a suitable alkylchlorocarbonate with 4-amino-1-methyl-3-n.propylpyrazolo-5-carboxamide hydrochloride (III) allows to obtain 4-amino-1-methyl-3-n.propylpyrazolo-5-carboxamide hydrochloride (VI) in a pure form and with a very good yield, up to 95%: the same compound is obtained, e.g., as indicated in Pfizer USP n°5,250,534, Example 6, with a yield of 40%. Further the preparation of the hydrochloride (III) has been realized using an original methodology based on the catalytic hydrogenation at low pressure in the presence of nickel-Raney in ethylacetate of the corresponding nitro compound (X), which enables, after removal of the catalyst, to have the amino hydrocloride (III) in a extremely high pure degree and high yield by simple acidification with aqueous hydrochloric acid. Thus the overall yield of (VI), using as starting material the benzoic acid derivative (IX) can be as high as 95%.

Typically, intermediate (VI) is added to ice-cooled chlorosulphonic acid at a temperature below 25°C, then reaction is allowed to continue at room temperature until completed. The reaction mixture is then poured into cold water and the wet cake of pure (V) can be directly treated, after dissolution in CH₂Cl₂, with 1-methylpiperazine to give the corresponding sulphonamide derivative (II). This same compound was prepared, e.g. through a less economical procedure, in EP-A-0812845 by amidation of the active imidazolide of 2-ethoxy-5-(4-methylpiperazin-1-ylsulphonyl)benzoic acid in a 72 hours coupling reaction with an ethylacetate solution of 4-amino-1-methyl-3-n.propylpyrazole-5-carboxamide.

Thus the overall yield of the sulphonamide derivative (II), using the intermediate (VI) and in accordance with the above described method, can be as hight as 86%.

The final cyclization reaction of the sulphonamide derivative (II) was carried out in a suitable alcohol in the presence of an ammonium quaternary base such as an alkylammonium hydroxide, preferably in 1,3-butanediol in the presence of 3.3 moles equivalents of tetrabutylammonium hydroxide. The tetrabutylammonium hydroxide can be prepared in situ from the corresponding bromide with the stoichiometric amount of sodium hydroxide, and provided, with a yield of 95%, the desired sildenafil compound (I) having a 99% purity degree. The cyclization reaction is carried out at from 100 to 110°C for 10 minutes; isolation of the final compound (I) is performed by neutralization of the pre-diluted reaction mixture, followed by separation of the product by filtration. In alternative, sildenafil (I) can be obtained without isolation of any intermediate starting from the compound of formula (VI) through the following described sequence: 1) chlorosulphonation of (VI); 2) anhydrification by dissolution in acetonitrile and subsequent azeotropic distillation of the solvent; 3) dissolution of the anhydrous chlorosulphonyl derivative (V) in 1,3-butanediol containing tetrabutylammonium hydroxide and 1-methylpiperazine. When in HPLC the N-sulphonylation of 1-methylpiperazine is complete, the reaction mixture is heated to 100-110°C for 10 minutes, then allowed to cool to room temperature. Water is added and the obtained solution neutralized to pH 9.25. The precipitate collected by filtration gives sildenafil (I) with an overall yield of 81% based on (V), with the HPLC purity up to 98,5%.

A further alternative route is based on the easy accessibility of salicylic acid chloride (XIa) and aminopyrazole hydrochloride (III). The salicylic acid chloride (XIa), obtained as described in J.Med.Chem. 30,1321,1987 or by treatment of the corresponding acid with a chlorinating agent such as, for example, SOCl₂ or oxalyl chloride, is condensed with the aminopyrazole hydrochloride (III) in dichloromethane in the presence of a tertiary base to give, with satisfactory yields, the phenol derivative (IV), which is then submitted to O-ethylation with diethylsulfate in methylethylketone in the presence of a base, such as potassium carbonate, to give with excellent yields the ethylderivative (VI).

The above method appears to be economically advantageous in respect to the one described in EP-A-0 463 756 since it uses the cheaper salicylic acid and it does not require the use of condensing agents and it also avoids otheres problems as the availability of 2-ethoxybenzoic acid commonly used as starting material for the synthesis of sildenafil (I).

The above compound (VI) has been previously converted into the corresponding pirimidin-7-one (VII) for treatment with NaOH and H₂O₂ at 30% in ethanol/water (EP-A-0463756) with a yield of 72% after chromatographic purification on silicagel. The above cyclization can be obtained according to a more economical route, which provides yields as high as 95% and HPLC purity up to 99%, using tetrabutylammonium hydroxide in 1,3-butanediol, at a temperature of 100-110°C for 10-15 minutes.

The same method can be conveniently applied to obtain compound (VIII) previously obtained with poor yield (10%) by de-alkylation induced from complexes of Pd(0) from the corresponding alkylether (EP-A-0 463 756) as in (VI) description.

From the above clearly appear the advantages shown by the process of the invention when compared to the previously known processes for the preparation of sildenafil (I).

The intermediate compounds shown by formula (III), (IV), (V), and (VIII) are new and are encompassed within the invention.

### Example 1

### 4-Amino-1-methyl-3-n.propylpyrazole-5-carboxamide hydrochloride (III)

To a stirred suspension in 150ml ethylacetate of 15g (70.68 mmoles) 1-methyl-4-nitro-3-n.propylpyrazole-5-carboxamide, 5ml of a suspension of Nickel-Raney in water are added and the resulting mixture hydrogenated at 70psi for 8 hours. After the uptake of hydrogen is complete, the catalyst is removed by filtration, the filter washed with ethylacetate, the washings and filtrate combined together and hydrochloric acid added thereto. The precipitate is collected by filtration, washed with ethylacetate and dried under vacuum to give 14,85g 4-amino-1-methyl-3-n.propylpyrazole-5-carboxamide hydrochloride melting at 240-242°C. Yield 93.6%.

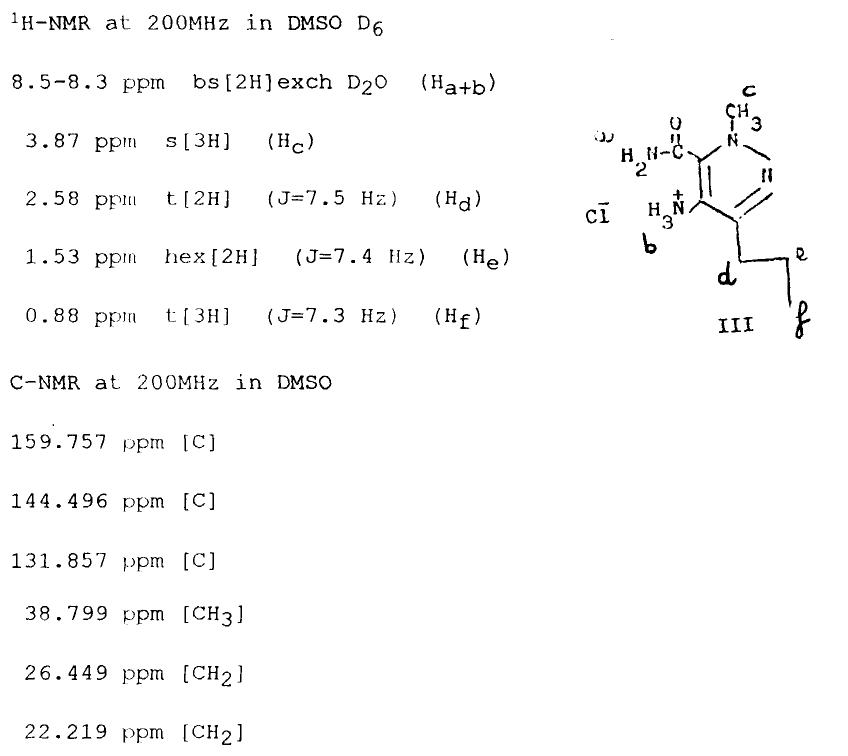

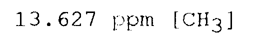

### Example 2

### 4-(2-Ethoxybenzamido)-1-methyl-3-n.propylpyrazole-5-carboxamide (VI)

To a solution of 42.6g 2-ethoxybenzoic acid (0.256 moles) in 430ml dichloromethane, at 0°C and under stirring, 78.5ml triethylamine (0.564 mol), 2ml N-methylmorfoline and 25.7ml ethylchloroformate (0.269 mol) are added, stirring is maintained for 30 minutes then 56g 4-amino-1-methyl-3-n.propylpyrazole-5-carboxamide hydrochloride (0.256 moles) are added in a single portion thereto.

The reaction mixture is kept under stirring overnight, the suspension washed with 100ml of 5% aqueous hydrochloric acid, 100ml water and 4% sodium bicarbonate. The organic phase is concentrated to dryness under vacuum and gives a residue which, crystallized from 200ml isopropyl ether, gives 80.35g 4-(2-ethoxybenzamido)-1-methyl-3-n.propylpyrazole-5-carboxamide melting at 154-155°C. Yield 95%

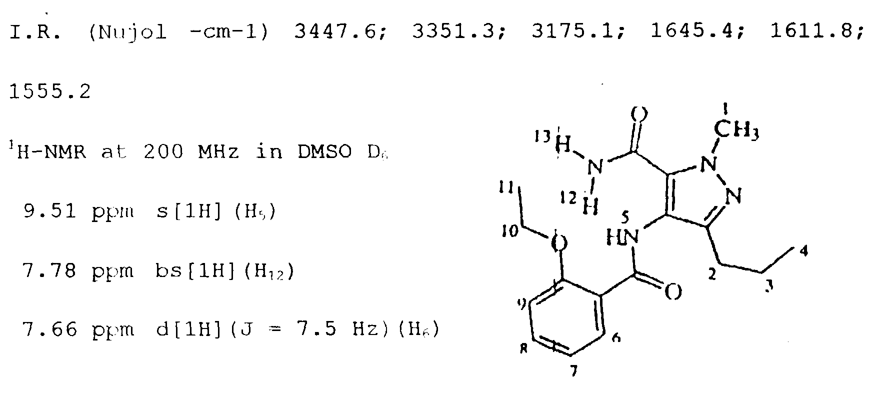

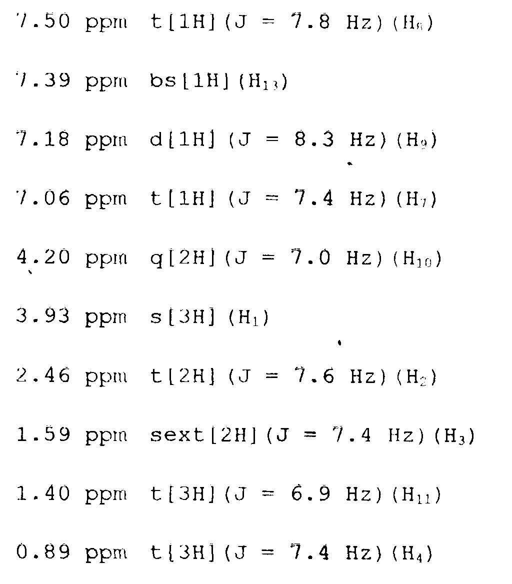

### Example 3

### 4-[2-Ethoxy-5-(4-methylpiperazin-1-ylsulphonyl)benzamido]-1-methyl-3-n.propylpyrazole-5-carboxamide (II)

To 40.42ml (0.605 moles) chlorosulphonic acid at 0°C under stirring 40g (0.121 moles) 4-(2-ethoxybenzamido)-1-methyl-3-n.propylpyrazole-5-carboxamide are added, temperature is brought to 35°C and maintained for 2 hours, then the dense solution is cooled to 0°C and poured into 1 kg ice flakes. The reaction mixture is stirred for a further hour at 0°C, the precipitate filtered under vacuum, the panel washed four times with 50ml each of water, pre-cooled to 4°C, to give 4-(5-chlorosulphonyl-2-ethoxybenzamido)-1-methyl-3-n.propylpyrazole-5-carboxamide (V), a sample of which dried under vacuum at 30°C melts at 153-155°C.

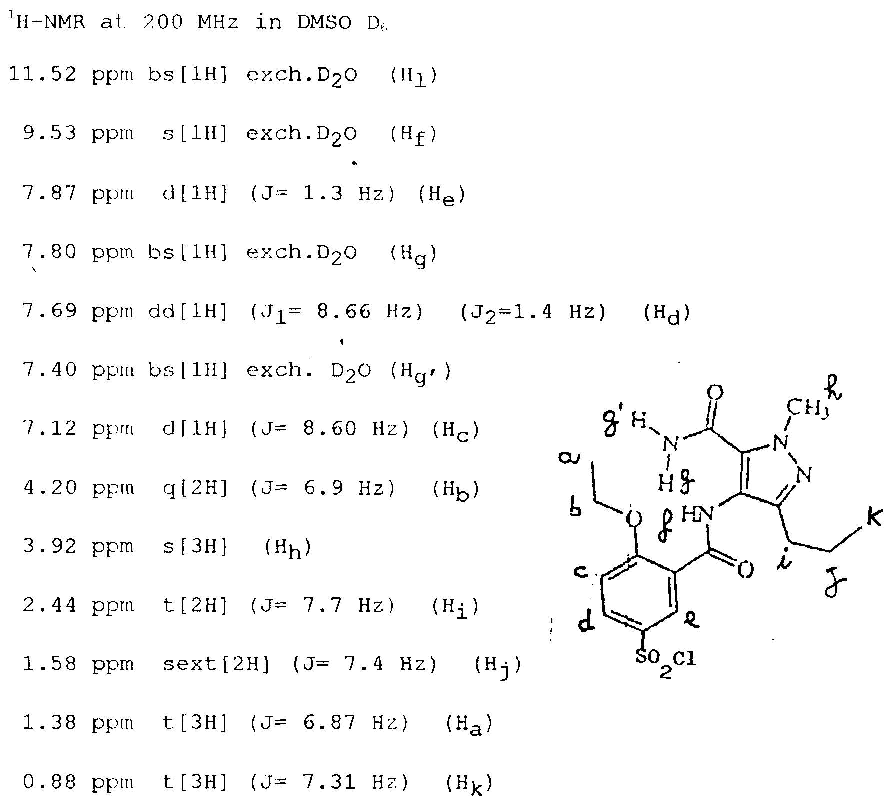

The obtained wet panel of 4-(5-chlorosulphonyl-2-ethoxybenzamido)-1-methyl-3-n.propylpyrazole-5-carboxamide is dissolved in 500ml dichloromethane and maintained under stirring for 15 minutes, then the aqueous phase is separated and the dichloromethylene solution treated with 36.36g(0.363 moles) 1-methylpiperazine. The solution is stirred at room temperature for one hour then 350ml water are added thereto, the reaction mixture acidified to pH 0.5 with concentrated hydrochloric acid and kept under stirring for 30 minutes. The phases are separated and the aqueous phase is decolorized with 2.5g charcoal, filtered and alcalized to pH 9.5 with 30% NaOH.

A white crystalline product precipitates which is filtered and dried under vacuum at 40°C to give 51.2g 4-[2-ethoxy-5-(4-methylpiperazin-1-ylsulphonyl)benzamido]-1-methyl-3-n.propyl-pyrazole-5-carboxamide melting at 204-205°C. Yield 86%.

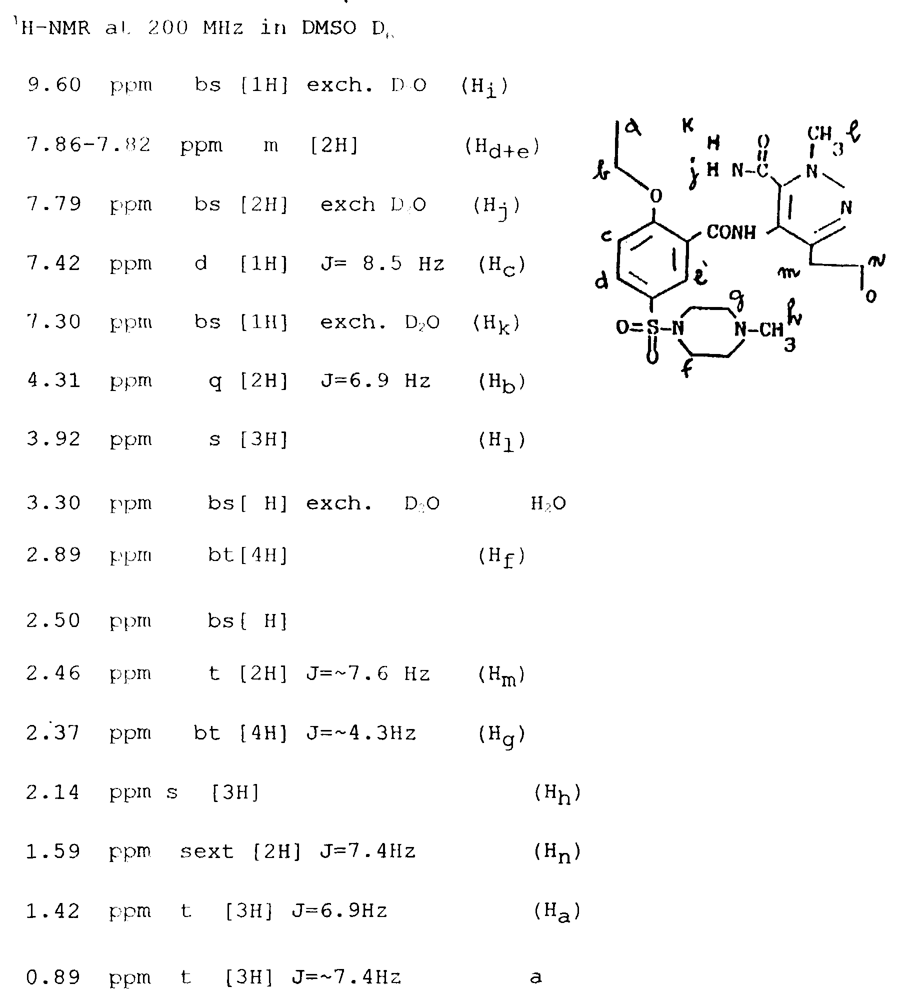

### Example 4

### 5-[2-Ethoxy-5-(4-methylpiperazin-1-ylsulphonyl)phenyl]-1-methyl-3-n.propyl-1,6-dihydro-7H-pyrazolo[4,3-d]pyrimidin-7-one (I)

To a solution of 8.12g (0.203 moles) NaOH in 20ml H₂O and 203ml 1,3-butanediol 65.57g (0.203 moles) tetrabutylammonium bromide are added, the mixture is kept under stirring for 30 minutes at 40°C then 30g (0.0609 moles) 4-[2-ethoxy-5-(4-methylpiperazin-1-ylsulphonyl)benzamido]-1-methyl-3-n.propylpyrazole-5-carboxy- amide are added thereto. The obtained solution is heated to 110°C for 10 minutes, then cooled to 10°C and poured in 11 water pre-cooled to 10°C. The solution is decolorized with 1.5g charcoal, filtered, and acidified to pH 9.25 with an aqueous solution (1:1) of hydrochloric acid. The precipitate is filtered, washed with water at 10°C and dried under vacuum at 40°C to give 27.45g 5-[2-ethoxy-5-(4-methylpiperazin-1-ylsulphonyl)phenyl]-1-methyl-3-n.propyl-1,6-dihydro-7H-pyrazolo [4,3-d]pyrimidin-7-one melting at 187-189°C. Yield 95%.

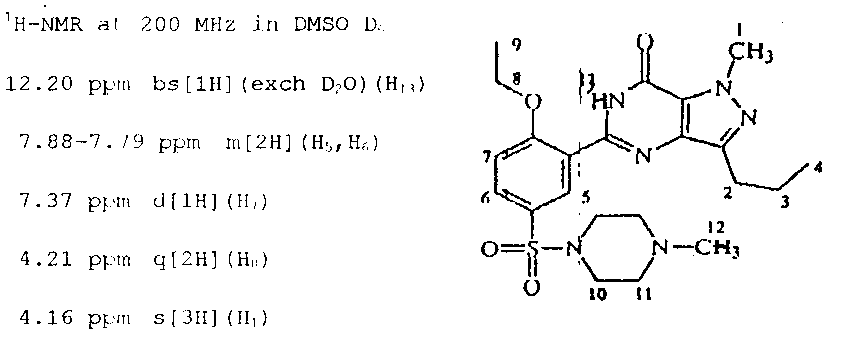

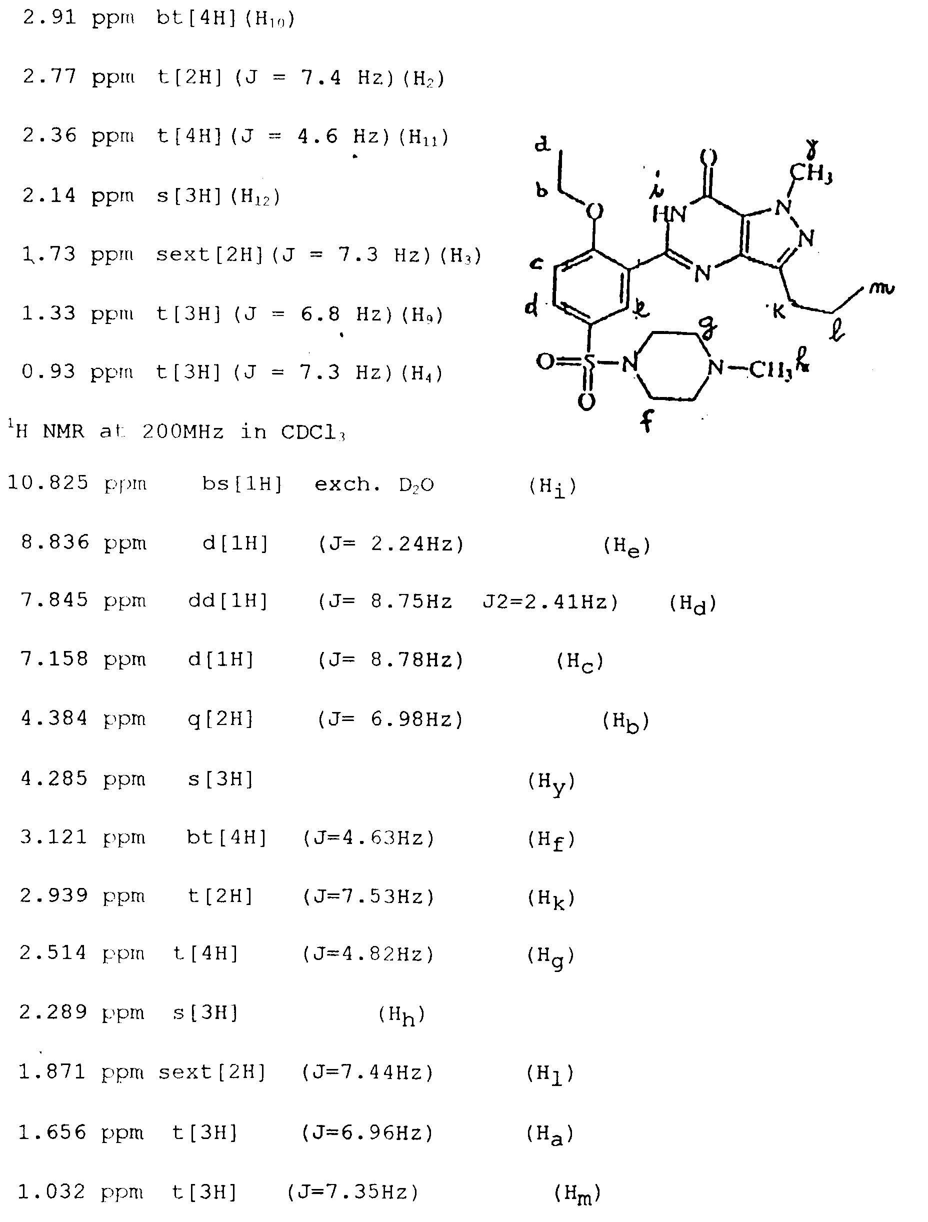

### Example 5

### 5-[2-Ethoxy-5-(4-methylpiperazin-1-ylsulphonyl)phenyl]-1- methyl-3-n.propyl-1,6-dihydro-7H-pyrazolo[4,3-d]pyrimidin-7-one (I) (one step procedure)

Grams 60 of 4-(5-chlorosulphonyl-2-ethoxybenzamido)-1-methyl-3-n.propylpyrazole-5-carboxamide wet panel (V), corresponding to 32.42g of dried product (0.0756 moles) prepared as described in Example 3, are taken up with 250ml acetonitrile and concentrated to dryness under vacuum at 40°C. The obtained residue is treated with 220ml of a 1,3-butanediol solution, containing 13.3g (0.332 moles) NaOH in 30ml water, 106.9g (0.332 moles) tetrabutylammonium bromide and 9.32g (0.08316 moles) 1-methylpiperazine. The reaction mixture is stirred at room temperature for 30 minutes then slowly heated to 110°C for 10 minutes, cooled to 10°C and poured into 11 water, pre-cooled to 10 °C. The solution, having pH 13, is decolorized with 2g charcoal, filtered,the pH adjusted to 9.25 with concentrated hydrochloric acid, the precipitate filtered under vacuum, washed on the filter with water pre-cooled to 10°C and dried to give 29.06g 5-[2-ethoxy-5-(4-methylpiperazin-1-ylsulphonyl)phenyl]-1-methyl-3-n.propyl-1,6-dihydro-7H-pyrazolo[4,3-d]pyrimidin-7-one melting at 187-189°C and showing the same NMR data as reported in Example 4. Yield 81%.

### Example 6

### 4-(2-Hydroxybenzamido-1-methyl-3-n.propylpyrazole-5-carboxamide (IV)

To a solution of 40g (0.255 moles) 2-hydroxybenzoylchloride (J. Chem.Am.Soc. 42, 604,1920) in 400ml dichloromethane are added under stirring at 0°C, first 56.77g (0.561 moles) triethylamine, then, in a single portion, 55.77g (0.255 moles) 4-amino-1-methyl-3-n.propylpyrazole-5-carboxamide hydrochloride. The reaction mixture is kept under stirring for 3 hours at 10°C, washed with 100ml solution of 5% hydrochloric acid, 100ml water and a solution of 4% sodium bicarbonate. The organic phase is concentrated to dryness under vacuum, the residue crystallized from 200ml isopropanol gives 71.69g 4-(2-hydroxybenzamido-1-methyl-3-n.propylpyrazole-5-carboxamide melting at 201-205°C. Yield 93%.

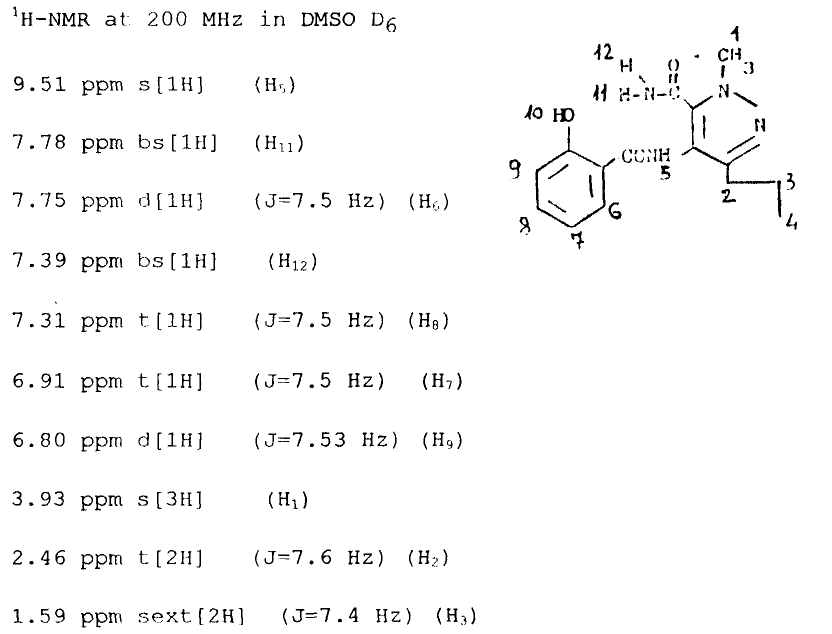

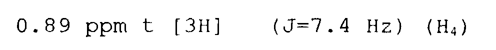

### Example 7

### 4-(2-Ethoxybenzamido)-1-methyl-3-n.propylpyrazole-carboxamide (VI)

In a flask equipped with a reflux condenser are placed 2g (6.62 mmoles) 4-(2-hydroxybenzamido-1-methyl-3-n.propylpyrazole-5-carboxamide, 1.03g (6.7 mmoles) diethylsulphate, 0.93g (6.7 mmoles) potassium carbonate and 25ml acetone. The mixture is refluxed for 10 hours, cooled, treated with 25ml water and extracted 3 times with 10ml each dichloromethane. The combined extracts are washed with a 10% aqueous NaOH solution and 3 times with 10 ml each NaCl solution, then dried to give 1.77g 4-(2-ethoxybenzamido)-1-methyl-3-n.propylpyrazole-carboxamide melting at 154-156°C. Identical by ¹H-NMR to the product prepared from product (X).

### Example 8

### 5-(2-Hydroxyphenyl)-1-methyl-3-n.propyl-1,6-dihydro-7H- pyrazolo[4,3-d]pyrimidin-7-one (VIII)

To a solution of 1g NaOH (0.025 moles) in 2.5ml water and 25ml 1,3-butanediol 8.06g (0.025 moles) tetrabutylammonium bromide are added. The reaction mixture is stirred at 40°C for 40 minutes then 2.27g (0.075 moles) 4-(2-hydroxybenzamido-1-methyl-3-n.propylpyrazole-5-carboxamide are added thereto and the solution obtained is heated to 110°C for 25 minutes, cooled to 10°C and poured in 100ml water pre-cooled to 10°C. The solution is cautiously acidified to pH 7 with a 15% aqueous hydrochloridric acid solution, the precipitate separated by filtration under vacuum and washed with cold water, dried under vacuum at 50°C to give 1.81g 5-(2-hydroxyphenyl)-1-methyl-3-n.propyl-1,6-dihydro-7H-pyrazolo[4,3-d]pyrimidin-7-one melting at 230-235°C. Yield 85%.

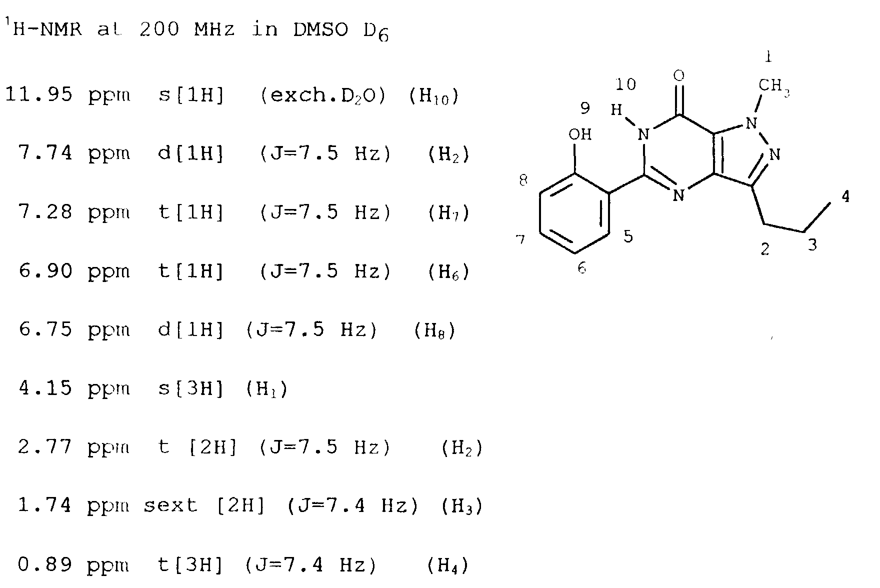

### Example 9

### 5-(2-Ethoxyphenyl)-1-methyl-3-n.propyl-1,6-dihydro-7H-pyrazolo[4,3-d]pyrimidin-7-one (VII)

To a solution of 4g (0.1 mole) NaOH in 10ml water and 100ml 1,3-butanediol, 32.23g (0.1 mole) tetrabutylammonium bromide are added, the temperature is brought to 40°C and the mixture stirred for 30 minutes, then 33.04g (0.1 mole) 4-(2-ethoxybenzamido)-1-methyl-3-n.propylpyrazole-5-carboxamide is added thereto and the mixture heated to 110°C for 15 minutes, cooled to 10°C and poured into 500ml water pre-cooled to 10°C. The solution (pH=13) is acidified with concentrated hydrochloric acid to pH 1.5, maintained under stirring at 10°C for 1 hour, filtered under vacuum, the panel which forms washed at 5°C five times with 20ml each water and dried under vacuum at 40°C to give 29g 5-(2-ethoxyphenyl)-1-methyl-3-n.propyl-1,6-dihydro-7H-pyrazolo[4,3-d]pyrimidin-7-one melting at 143-146°C.

## Claims

1. A process for the preparation of the compound of formula (I) 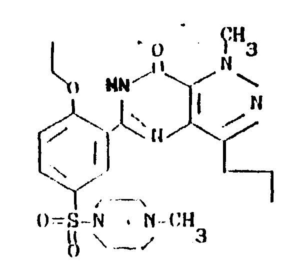 which comprises reacting a mixed anhydride of 2-ethoxybenzoic acid (IXa) with 4-amino-1-methyl-3-n.propylpyrazole-5-carboxamide hydrochloride (III) in the presence of triethylamine and N-methylmorfoline at 0°C to obtain 4-(2-ethoxybenzamido)-1-methyl-3-n.propylpyrazole-5-carboxamide(VI) which is reacted with chlorosulphonic acid at a temperature below 25°C then at room temperature to give 4-(5-chlorosulphonyl-2-ethoxybenzamido)-1-methyl-3-n.propylpyrazole-5-carboxamide (V) which, without being isolated from the reaction mixture is treated, suspended in water, with 1-methylpiperazine to give 4-[2-ethoxy-5-(4-methylpiperazin-1-ylsulphonyl)-benzamido]-1-methyl-3-n.propylpyrazole-5-carboxamide (II) which is cyclized in a suitable alcohol in the presence of an ammonium quaternary base at the temperature of from 100 to 110°C to give the desired 5-[2-ethoxy-5-(4-methylpiperazin-1-ylsulphonyl)-phenyl]-1-methyl-3-n.propyl-1,6-dihydro-7H-pyrazolo-[4,3- d]pyrimidin-7-one (I).

2. The process according to claim 1, wherein 4-[2-ethoxy-5-(4-methylpiperazin-1-ylsulphonyl)benzamido] -1-methyl-3-n.propylpyrazole-5-carboxamide (II) is cyclized in 1,3-butanediol in the presence of 3.3 mole equivalents of tetrabutylammonium hydroxide.

3. The process according to claim 2, wherein 4-(2-ethoxybenzamido)-1-methyl-3-n.propylpyrazole-5-carboxamide (VI) is submitted to a one-step reaction consisting of chlorosulphonation followed by anhydrification in acetonitrile and subsequent distillation of the solvent and the anhydrous 4-(5-chlorosulphonyl-2-ethoxybenzamido)-1-methyl-3-n.propylpyrazole-5-carboxamide (V) is dissolved in 1,3-butanediol and tetrabutylammonium hydroxide and 1-methylpiperazine, at a temperature of from 100 to 110°C, to give the desired 5-[2-ethoxy-5-(4-methylpiperazin-1-ylsulphonyl)phenyl]-1-methyl-3-n.propyl-1,6-dihydro-7H-pyrazolo[4,3-d]pyrimidin-7-one (I).

4. The process according to claim 1, wherein 4-(2-ethoxybenzamido)-1-methyl-3-n.propylpyrazole-5-carboxamide (VI) is prepared starting from salicylic acid chloride (XIa) which is condensed in dichloromethane in the presence of a tertiary base with 4-amino-1-methyl-3-n.propylpyrazole-5-carboxamide hydrochloride (III) to give 4-(2-hydroxybenzamido)-1-methyl-3-n.propylpyrazole-5-carboxamide (IV), which is then submitted to O-ethylation with diethylsulfate in methylethylketone in the presence of a base, to give 4-(2-ethoxybenzamido)-1-methyl-3-n.propylpyrazole-5-carboxamide (VI).

5. The process according to claim 1, wherein 4-amino-1-methyl-3-n.propylpyrazole-5-carboxamide hydrochloride (III) is obtained by means of catalytic hydrogenation at low pressure in the presence of nickel-Raney in ethylacetate of 1-methyl-4-nitro-3-n.propylpyrazole-5-carboxamide (X) followed by acidification with aqueous hydrochloric acid.

6. 4-amino-1-methyl-3-n.propylpyrazole-5-carboxamide hydrochloride (III).

7. 4-(2-hydroxybenzamido)-1-methyl-3-n.propylpyrazole-5-carboxamide (IV).

8. 4-(5-chlorosulphonyl-2-ethoxybenzamido)-1-methyl-3-n.propylpyrazole-5-carboxamide (V).

9. 5-(2-Hydroxyphenyl)-1-methyl-3-n.propyl-1,6-dihydro-7H-pyrazolo[4,3-d]pyrimidin-7-one (VIII).
